(19) Europäisches Patentamt
European Patent Office
Office européen des brevets

(11) Publication number: **0 250 037 B1**

# (12) EUROPEAN PATENT SPECIFICATION

(45) Date of publication of patent specification: **04.09.91**

(51) Int. Cl.$^5$: **C07C 273/18**, C07C 275/28, B01J 31/04

(21) Application number: **87201108.5**

(22) Date of filing: **11.06.87**

## (54) Process for the preparation of urea derivatives and catalyst system.

(30) Priority: **20.06.86 GB 8615155**

(43) Date of publication of application:
**23.12.87 Bulletin 87/52**

(45) Publication of the grant of the patent:
**04.09.91 Bulletin 91/36**

(84) Designated Contracting States:
**BE CH DE FR GB LI NL**

(56) References cited:
**EP-A- 0 086 281**
**WO-A-85/01285**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Drent, Eit**
**Badhuisweg 3**
**NL-1031 CM Amsterdam(NL)**

(74) Representative: **Tuijn, Jan Warnaar et al**
**Shell Internationale Research Maatschappij B.V., Patents, Licensing & Trade Marks Division, P.O. Box 302**
**NL-2501 CH The Hague(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The invention relates to a process for the preparation of urea derivatives, to the urea derivatives prepared and to a catalyst system, used in the preparation of the urea derivatives.

The invention particularly relates to the preparation of urea derivatives of the general formula:

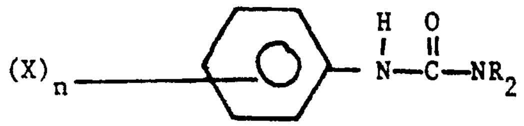

(I)

wherein R is an alkyl group and X is a halogen atom, an alkyl-, alkoxy-, aryloxy-, cyano-, ester- or trifluoromethyl group and n is from 0 to 5.

It is generally known that the urea derivatives may be prepared by reaction of isocyanates with amines. Isocyanates may be prepared via the well-known phosgen route. Isocyanates and phosgen are known as very poisonous.

Applicant has now found a one-step process, in which the use of the poisonous phosgen is avoided and isocyanates do not need to be isolated.

The invention relates to a process for the preparation of urea derivatives of the general formula

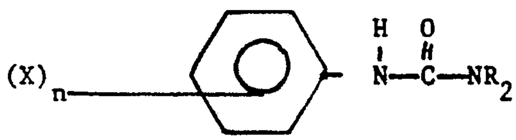

(I)

wherein R is an alkyl group and X is a halogen atom, an alkyl-, alkoxy-, aryloxy-, cyano-, ester- or trifluoromethyl group and n is from 0 to 5,
which comprises reacting an aromatic nitro compound of the general formula

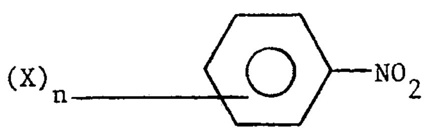

(II)

wherein X and n are as defined above with carbon monoxide and an amine $HNR_2$ wherein R is an alkyl group,
in the presence of a catalyst system comprising

(a) a group VIII noble metal or a compound thereof,

(b) a bidentate ligand of the formula

$R^1R^2$-M-A-M-$R^3R^4$

in which M is P, As or Sb, A is a divalent organic bridging group having at least 2 carbon atoms in the bridge, none of these carbon atoms carrying substituents that may cause steric hindrance, and $R^1$ ,$R^2$ ,$R^3$ and $R^4$ represent similar or dissimilar optionally substituted hydrocarbon groups, and

(c) an acid having a pKa of more than 2 or a transition metal salt of said acid.

In the US patent specifications 3,523,962; 3,903,125 and 3,914,268 is disclosed the preparation of an aromatic isocyanate by reacting an aromatic nitro compound with carbon monoxide with the aid of a noble metal catalyst, but mention of urea compounds has not been made.

A process for preparing carbamates is disclosed by EP-A-86281, wherein an organic nitrogenous compound, an organic hydroxyl containing compound, and carbon monoxide are reacted in the presence of a catalyst system based on palladium and a bifunctional Group VA ligand. The catalyst system may further comprise an acid. Symmetric urea derivatives may be initially formed or be added as reactant in advance, but appear to be converted to carbamates in this known process. WO-A-85/01285 discloses a process for converting a nitrogen-containing compound into the corresponding urethane in the presence of a catalyst

system comprising a halide-free ruthenium compound. No mention of any use of an acid is made. Again, symmetric urea derivatives may be initially formed, but are ultimately converted in other product in this known process.

The urea derivatives prepared by the process according to the invention may be substituted or unsubstituted in the phenyl group in formula (I). X may be an alkyl group, preferably having from 1 to 10 carbon atoms, more preferably having from 1 to 4 carbon atoms. When X is halogen, chlorine and bromine are preferred. Preferably the alkoxy and aryloxy groups comprise not more then 10 carbon atoms and are preferably bound to the phenyl nucleus via the oxygen atom. The ester group preferably has not more then 10 carbon atoms, more preferably not more then 6 carbon atoms. The most preferred substituents are chlorine atoms and methyl groups.

In formula (I) the groups R are preferably alkyl groups having from 1 to 10 carbon atoms, more preferably having from 1 to 4 carbon atoms, such as methyl or ethyl groups.

As already indicated the substituents X in formula I and II correspond with each other. Preferred aromatic nitro compounds are nitrobenzene, 2-chloro-4-nitro toluene and para-isopropyl nitrobenzene.

The amines used as starting materials in the process according to the invention are preferably dialkylamines with alkyl groups having from 1 to 10 carbon atoms, more preferably having from 1 to 4 carbon atoms, such as dimethylamine and diethylamine.

The reaction is presumed to occur according to the following equation

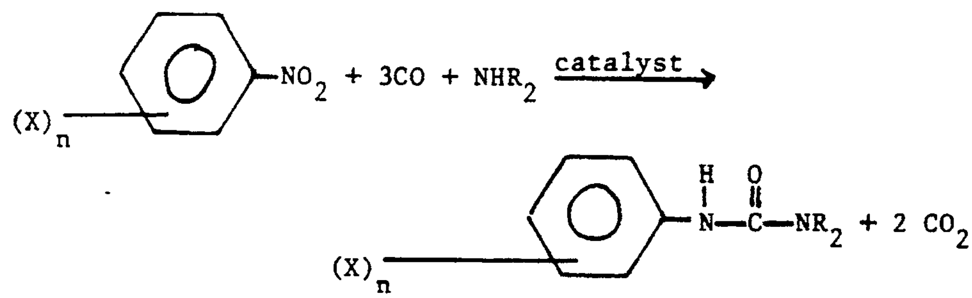


The reaction temperature may range from 50 °C to 200 °C, preferably from 70 °C to 150 °C. Pressures may range from atmospheric pressure to 200 bar.

The invention further relates to a catalyst system comprising

(a) a group VIII noble metal or a compound thereof,

(b) a bidentate ligand of the formula

$R^1R^2$-M-A-M-$R^3R^4$

in which M is P, As or Sb, A is a divalent organic bridging group having at least 2 carbon atoms in the bridge, none of these carbon atoms carrying substituents that may cause steric hindrance, and $R^1$,$R^2$,$R^3$ and $R^4$ represent similar or dissimilar optionally substituted hydrocarbon groups, and

(c) an acid having a pKa of more than 2 or a transition metal salt of said acid.

The noble metals of group VIII are palladium, ruthenium, rhodium, platinum, iridium and osmium. Palladium, platinum and rhodium are preferred, palladium being the most preferred. It is with reference to palladium compounds that hereinafter the process according to the invention is described in more detail.

Both homogeneous and heterogeneous palladium compounds can be used. Homogeneous systems are preferred. Suitable palladium compounds are salts of palladium with, for example, nitric acid, sulphuric acid or alkanoic acids containing not more than 12 carbon atoms per molecule. Salts of hydrohalogenic acids can in principle be used as well. Palladium carboxylates are the preferred catalyst compounds, in particular palladium acetate. Further, palladium acetylacetonate can also be used. Palladium on carbon and palladium combined with an ion exchanger are examples of suitable heterogeneous palladium compounds.

The quantity of palladium, or other noble metal or compound thereof is not critical. Preference is given to the use of quantities in the range of from $10^{-4}$ to $10^{-1}$ mol per mol of aromatic nitro compound.

In the bidentate ligand substituents offering steric hindrance should be absent, which means that no substituents may be present that are able to hinder the formation of complex compounds having the general formula

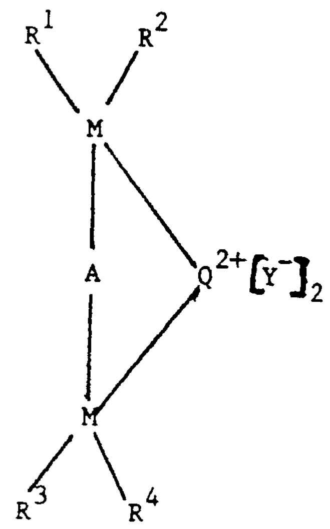

In that formula, Q represents Pd or other noble metal, Y represents a non-coordinating anion, whilst $Q^{2+}$ can also be written as

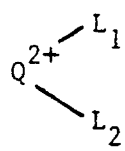
,

in which the ligands $L_1$ and $L_2$ are weakly coordinated solvent ligands, e.g. acetonitrile, methanol, acetone, or acetylacetone, or correspond with those employed in the palladium compounds described in the preceding paragraph.

In the bidentate ligand, M is preferably phosphorus. Hydrocarbon groups $R^1$, $R^2$, $R^3$ and $R^4$ will as a rule contain 2 to 18 carbon atoms, preferably 6 to 14 carbon atoms. Aryl groups are the most suitable, in particular the phenyl groups. Preferred bridging groups -R- are those having the formula $(CR^5R^6)$ in which $R^5$ and $R^6$ are hydrogen atoms or hydrocarbon groups offering no steric hindrance and n is 3 or 4. Substituents $R^5$ and $R^6$ are preferably hydrogen atoms. The bridging groups R may also make part of cyclic structure, e.g. an aromatic or cycloaliphatic group, the carbon to carbon bond or bonds in the bridge may be saturated or unsaturated and in the bridge or in the cyclic or non-cyclic groups attached to the bridge one or more hetero atoms, e.g. sulphur, oxygen, iron or nitrogen, may have been substituted for carbon atoms, other than the two carbon atoms which must be present in the bridge linking both atoms M.

Examples of suitable bidentate ligands are
1,3-di(diphenylphosphino)propane,
1,4-di(diphenylphosphino)butane,
2,3-dimethyl-1,4-di(diphenylphosphino)butane,
1,4-di(dicyclohexylphosphino)butane,
1,3-di(di-p-tolylphosphino)propane,
1,4-di(di-p-methoxyphenylphosphino)butane,
2,3-di(diphenylphosphino)-2-butene,
1,3-di(diphenylphosphino)-2-oxopropane,
2-methyl-2-(methyldiphenylphosphino)-1,3-di(diphenylphosphino)propane,
0,0′-di(diphenylphosphino)biphenyl,
1,2-di(diphenylphosphino)benzene,
2,3-di(diphenylphosphino)naphthalene,
1,2-di(diphenylphosphino)cyclohexane,
2,2-dimethyl-4,5-di(diphenylphosphino)dioxolane and

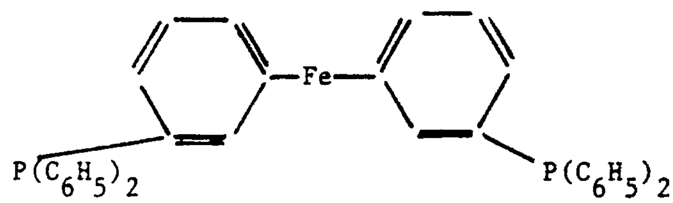

It is observed that compounds having a structure like 2-methyl-2-(methyldiphenylphosphino)-1,3-di-(diphenylphosphino)-propane, $CH_3$-C-($CH_2$-P($C_6H_5$)$_2$)$_3$, although being trifunctional, are nevertheless considered bidentate ligands in the terms of this invention since only two of the three phosphorous atoms can coordinate with the palladium atom in the complex.

The bidentate ligand can be employed in quantities which may vary within wide limits, e.g. of from 0.1 to 10 mol per mol of palladium or other noble metal.

The acid having a pKa of more than 2 is preferably a carboxylic acid having 2 to 14 carbon atoms, e.g. acetic acid, 9-anthracene carboxylic acid and 2,4,6-trimethylbenzoic acid.

The transition metal which may form a salt with the carboxylic acid, may be defined as a metal of group $I^B$, $II^B$, $III^B$, $IV^B$, $V^B$, $VI^B$ and VIII, the lanthanides and actinides of the Periodic System. Preferred are copper, iron and nickel. Iron and nickel salts give about the same conversion and selectivity as those with copper salts. It is observed that with a potassium salt no conversion at all is achieved.

The process according to the invention can be carried out conveniently in the presence of a solvent. A variety of solvents can be applied such as ketones, e.g. acetone, methyl ethyl ketone, methyl isobutyl ketone, acethophenone and cyclohexanone; ethers, such as diethylene glycol dimethylether (also referred to as "diglyme"), anisole, diphenyl ether; aromatic compounds, such as benzene, toluene and the three xylenes; halogenated aromatic compounds, such as chlorobenzene and ortho-dichlorobenzene; halogenated paraffinic hydrocarbons, such as methylene chloride and carbon tetrachloride; paraffins such as hexane, heptane, cyclohexane, methylcyclohexane and iso-octane; nitriles, such as benzonitrile and acetonitrile and esters such as methyl benzoate.

The reaction may be carried out batch-wise; semi-continuous or continuously.

The urea derivatives may be useful as herbicides, plant growth regulators or as intermediates in the preparation of urethanes.

Examples

In a Hastelloy C autoclave provided with magnetic stirrer were introduced 0.1 mol of an aromatic nitro compound, 0.075 mol of an amine, 50 ml diethylene glycol dimethylether as reaction solvent and the catalyst system. The autoclave was pressurized with CO at 60 bar and the temperature was raised to the required reaction temperature by electrical means. After the required reaction time the pressure was dropped, the autoclave was gradually cooled and the reaction mixture was analyzed by NMR and GLC/MS to determine the amount of urea derivative.

In the following table all examples are given with the amounts of catalyst components in millimoles, the reaction times (t), the reaction temperatures (T), the conversion (calculated on the amine) and the selectivity to urea derivative, the amine and the aromatic nitro compound as starting compounds.

TABLE

| Example | Catalyst mmole | Catalyst compound | t h | T °C | amine | 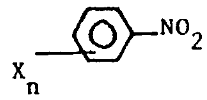 | conversion % | selectivity to urea derivative |
|---|---|---|---|---|---|---|---|---|
| 1 | 0.1<br>1<br>10 | $Pd(OAc)_2$<br>$\phi_2-P-(CH_2)_3-P-\phi_2$<br>H(OAc) | 1 | 110 | $HN(C_2H_5)_2$ | nitrobenzene | 95 | 44 |
| 2 | 0.1<br>2<br>10 | $Pd(OAc)_2$<br>$\phi_2-P-(CH_2)_3-P-\phi_2$<br>H(OAc) | 0.5 | 110 | $HN(C_2H_5)_2$ | nitrobenzene<br>1) (solvent) | 98 | 57 |
| 3 | 0.1<br>2<br>10 | $Pd(OAc)_2$<br>$\phi_2-P-(CH_2)_3-P-\phi_2$<br>H(OAc) | 5 | 110 | $HN(CH_3)_2$ | 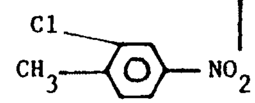 | 100 | 50 |
| 4 | 0.1<br>1<br>3 | $Pd(OAc)_2$<br>$\phi_2-P-(CH_2)_3-P-\phi_2$<br>$Cu(OAc)_2$ | 5 | 80 | $HN(C_2H_5)_2$ | nitrobenzene | 95 | 53 |
| 5 | 0.1<br>1<br>10 | $Pd(OAc)_2$<br>$\phi_2-P-(CH_2)_3-P-\phi_2$<br>H(OAc) | 0.5 | 110 | $HN(CH_3)_2$ | 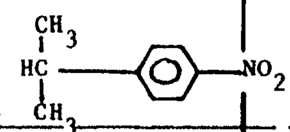 | 80 | 35 |
| 6 | 0.1<br>1<br>3 | $Pt(Acac)_2$<br>$\phi_2-P-(CH_2)_3-P-\phi_2$<br>$Cu(OAc)_2$ | 5 | 110 | $HN(C_2H_5)_2$ | nitrobenzene | 40 | 25 |
| 7 | 0.1<br>1<br>3 | $Ru(Acac)_3$<br>$\phi_2-P-(CH_2)_3-P-\phi_2$<br>$Cu(OAc)_2$ | 5 | 110 | $HN(C_2H_5)_2$ | nitrobenzene | 30 | 30 |

TABLE CONTINUED

| | | | | | | | | |
|---|---|---|---|---|---|---|---|---|
| 8 | 0.1<br>1<br>3 | Rh(Acac)(CO)$_2$<br>$\phi_2$-P-$(CH_2)_3$-P-$\phi_2$<br>$Cu(OAc)_2$ | 5 | 110 | $HN(C_2H_5)_2$ | nitrobenzene | 80 | 70 |
| 9<br>(COMP) | 0.1<br>2<br>3 | Rh(Acac)$_3$<br>triphenylphosphine<br>$Cu(OAc)_2$ | 5 | 110 | $HN(C_2H_5)_2$ | nitrobenzene | 20 | 15 |
| 10<br>(COMP) | 0.1<br>2<br>3 | $Pd(OAc)_2$<br>triphenylphosphine<br>$Cu(OAc)_2$ | 5 | 80 | $HN(C_2H_5)_2$ | nitrobenzene | 20 | 5 |

Meaning of symbols in the table:

H(OAc) = acetic acid

$\phi_2$-P-$(CH_2)_3$-P-$\phi_2$ = 1,3-di(diphenylphosphino)propane

Acac = acetylacetonate

1) 50 ml diethylene glycol dimethylether replaced by 50 ml nitrobenzene

## Claims

1. A process for the preparation of urea derivatives of the general formula

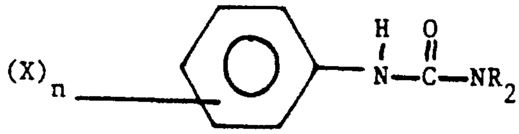 (I)

wherein R is an alkyl group and X is a halogen atom, an alkyl-, alkoxy-, aryloxy-, cyano-, ester- or trifluoromethyl group and n is from 0 to 5,
which comprises reacting an aromatic nitro compound of the general formula

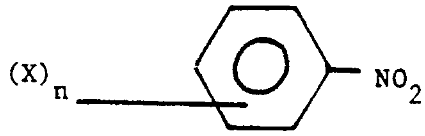 (II)

wherein X and n are as defined above with carbon monoxide and an amine $HNR_2$ wherein R is an alkyl group,
in the presence of a catalyst system comprising
(a) a group VIII noble metal or a compound thereof,
(b) a bidentate ligand of the formula

$R^1R^2$-M-A-M-$R^3R^4$

in which M is P, As or Sb, A is a divalent organic bridging group having at least 2 carbon atoms in the bridge, none of these carbon atoms carrying substituents that may cause steric hindrance, and $R^1$,$R^2$,$R^3$ and $R^4$ represent similar or dissimilar optionally substituted hydrocarbon groups, and
(c) an acid having a pKa of more than 2 or a transition metal salt of said acid.

2. A process according to claim 1 wherein the amine $HNR_2$ has R groups having from 1 to 4 carbon atoms.

3. A process according to claim 2 wherein the amine is dimethylamine or diethylamine.

4. A process according to one or more of the claims 1-3 wherein the aromatic nitro compound has at least one alkyl group of 1 to 4 carbon atoms or a chlorine atom.

5. A process according to claim 4 wherein the aromatic nitro compound is p-isopropyl nitrobenzene; 3,4-dichloro nitrobenzene; 3-chloro-4-methyl nitrobenzene.

6. A process according to claim 1 wherein the aromatic nitro compound is nitrobenzene.

7. A process according to one or more of the claims 1-6 wherein the group VIII noble metal is palladium, ruthenium, rhodium, platinum or iridium.

8. A process according to one or more of the claims 1-7 wherein a bidentate phosphine is used.

9. A process according to claim 8 wherein the bidentate phosphine is 1,3-di(diphenylphosphino)propane.

10. A process according to one or more of the claims 1 and 7-9 wherein the acid is a carboxylic acid, having 2 to 10 carbon atoms.

11. A process according to claim 10, wherein the carboxylic acid is acetic acid.

12. A process according to claim 1, 10 or 11, wherein the salt of the transition metal is a copper salt, an iron salt or a nickel salt.

13. A process according to any one of the preceding claims wherein the reaction takes place in the

presence of a solvent.

**14.** A process according to claim 13 wherein the solvent is diethylene glycol dimethylether or nitrobenzene.

**15.** A process according to any one of the preceding claims wherein the reaction temperature is between 50 °C and 200 °C, preferably between 70 °C and 150 °C.

**16.** A catalyst system comprising

(a) a group VIII noble metal or a compound thereof,

(b) a bidentate ligand of the formula

$R^1R^2$-M-A-M-$R^3R^4$

in which M is P, As or Sb, A is a divalent organic bridging group having at least 2 carbon atoms in the bridge, none of these carbon atoms carrying substituents that may cause steric hindrance, and $R^1$,$R^2$ ,$R^3$ and $R^4$ represent similar or dissimilar optionally substituted hydrocarbon groups, and

(c) a copper, nickel or iron salt of an acid having a pKa of more than 2.

**17.** A catalyst system according to claim 16, wherein the group VIII noble metal is palladium, ruthenium, rhodium, platinum or iridium.

**18.** A catalyst system according to claim 16 or 17 wherein in the formula of the bidentate ligand $R^1$ ,$R^2$ ,$R^3$ and $R^4$ are phenyl groups and M is a phosphorus atom, and A is a divalent straight alkyl chain.

**19.** A catalyst system according to one or more of the claims 16 to 18 wherein the acid is a carboxylic acid having 2 to 10 carbon atoms.

**Revendications**

**1.** Procédé de préparation de dérivés d'urée de formule générale

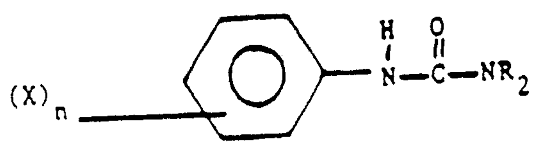

(I)

où R est un groupe alcoyle et X est un atome d'halogène, un groupe alcoyle, alcoxy, aryloxy, cyano, ester ou trifluorométhyle et n vaut de 0 à 5,
dans lequel on fait réagir un composé nitro aromatique de formule générale

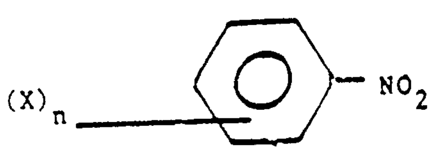

(II)

où X et n sont tels que définis ci-dessus, avec du monoxyde de carbone et une amine $HNR_2$ où R est un groupe alcoyle,
en présence d'un système catalyseur comprenant

(a) un métal noble du groupe VIII ou un de ses composés,

(b) un coordinat bidenté de formule

$R^1R^2$-M-A-M-$R^3R^4$

où M représente P, As ou Sb, A est un groupe de pontage organique bivalent ayant au moins 2 atomes de carbone dans le pont, aucun de ces atomes de carbone ne portant des substituants qui peuvent provoquer un empêchement stérique, et $R^1$, $R^2$, $R^3$ et $R^4$ représentent des groupes

hydrocarbonés éventuellement substitués semblables ou dissemblables, et,
(c) un acide ayant un pKa supérieur à 2 ou un sel de métal de transition dudit acide.

2. Procédé selon la revendication 1, où l'amine $HNR_2$ a des groupes R ayant de 1 à 4 atomes de carbone.

3. Procédé selon la revendication 2, où l'amine est la diméthylamine ou la diéthylamine.

4. procédé selon une ou plusieurs des revendications 1-3, où le composé nitro aromatique a au moins un groupe alcoyle en $C_{1-4}$ ou un atome de chlore.

5. Procédé selon la revendication 4, dans lequel le composé nitro aromatique est le p-isopropylnitrobenzène ; 3,4-dichloro-nitrobenzène ; 3-chloro-4-méthyl-nitrobenzène.

6. Procédé selon la revendication 1, dans lequel le composé nitro aromatique est le nitrobenzène.

7. Procédé selon une ou plusieurs des revendications 1-6 dans lequel le métal noble du groupe VIII est le palladium, le ruthénium, le rhodium, le platine ou l'iridium.

8. Procédé selon une ou plusieurs des revendications 1-7, dans lequel on utilise une phosphine bidentée.

9. Procédé selon la revendication 8, dans lequel la phosphine bidentée est le 1,3-di-(diphénylphosphino)-propane.

10. Procédé selon une ou plusieurs des revendications 1 et 7-9, dans lequel l'acide est un acide carboxylique ayant de 2 à 10 atomes de carbone.

11. Procédé selon la revendication 10, dans lequel l'acide carboxylique est l'acide acétique.

12. Procédé selon l'une des revendications 1, 10 ou 11, dans lequel le sel du métal de transition est un sel de cuivre, un sel de fer ou un sel de nickel.

13. Procédé selon l'une quelconque des revendications précédentes dans lequel la réaction se produit en présence d'un solvant.

14. Procédé selon la revendication 13, dans lequel le solvant est le diméthyléther de diéthylène-glycol ou le nitrobenzène.

15. Procédé selon l'une quelconque des revendications précédentes, dans lequel la température de la réaction est comprise entre 50°C et 200°C, de préférence entre 70°C et 150°C.

16. Système catalyseur comprenant
(a) un métal noble du groupe VIII ou un de ses composés,
(b) un coordinat bidenté de formule

$R^1R^2\text{-M-A-M-}R^3R^4$

où M représente P, As ou Sb, A est un groupe de pontage organique bivalent ayant au moins deux atomes de carbone dans le pont, aucun de ces atomes de carbone ne portant des substituants qui peuvent provoquer un empêchement stérique, et $R^1$, $R^2$, $R^3$ et $R^4$ représentent des groupes hydrocarbonés éventuellement substitués semblables ou dissemblables, et
(c) un sel de cuivre, nickel ou fer, d'un acide ayant un pKa supérieur à 2.

17. Système catalyseur selon la revendication 16, dans lequel le métal noble du groupe VIII est le palladium, le ruthénium, le rhodium, le platine ou l'iridium.

18. Système catalyseur selon la revendication 16 ou 17, dans lequel dans la formule du coordinat bidenté, $R^1$, $R^2$, $R^3$ et $R^4$ sont des groupes phényle et M est un atome de phosphore, et A est une chaîne

alcoyle droite bivalente.

**19.** Système catalyseur selon une ou plusieurs des revendications 16 à 18, dans lequel l'acide est un acide carboxylique ayant de 2 à 10 atomes de carbone.

**Patentansprüche**

**1.** Verfahren zur Herstellung von Harnstoffderivaten der allgemeinen Formel

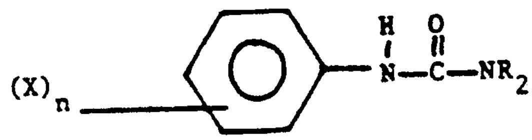

(I)

in der R eine Alkylgruppe ist und X für ein Halogen-Atom, eine Alkyl-, Alkoxy-, Aryloxy-, Cyano-, Ester- oder Trifluormethylgruppe steht und n eine Zahl von 0 bis 5 ist,
dadurch **gekennzeichnet,** daß man eine aromatische Nitroverbindung der allgemeinen Formel

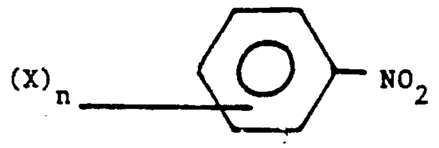

(II)

in der X und n wie oben definiert sind, mit Kohlenmonoxid und einem Amin $HNR_2$, worin R eine Alkylgruppe bedeutet, in Gegenwart eines Katalysatorsystems umsetzt, welches
(a) ein Edelmetall der Gruppe VIII oder eine Verbindung davon,
(b) einen zweizähnigen Liganden der Formel

$R^1R^2\text{-M-A-M-}R^3R^4$

in der M für P, As oder Sb steht, A eine zweiwertige organische brückenbildende Gruppe mit mindestens 2 Kohlenstoffatomen in der Brücke ist, wobei keines dieser Kohlenstoffatome Substituenten trägt, die sterische Hinderungen verursachen können, und $R^1$, $R^2$, $R^3$ und $R^4$ gleiche oder verschiedenen gegebenenfalls substituierte Kohlenwasserstoffgruppen bedeuten, und
(c) eine Säure mit einem pKa von mehr als 2 oder ein Übergangsmetallsalz dieser Säure,
umfaßt.

**2.** Verfahren nach Anspruch 1, worin das Amin $HNR_2$ R-Gruppen mit 1 bis 4 Kohlenstoffatomen aufweist.

**3.** Verfahren nach Anspruch 2, worin das Amin Dimethylamin oder Diethylamin ist.

**4.** Verfahren nach einem oder mehreren der Ansprüche 1 bis 3, worin die aromatische Nitroverbindung mindestens eine Alkylgruppe mit 1 bis 4 Kohlenstoffatomen oder ein Chloratom aufweist.

**5.** Verfahren nach Anspruch 4, worin die aromatische Nitroverbindung p-Isopropylnitrobenzol; 3,4-Dichlornitrobenzol oder 3-Chlor-4-methylnitrobenzol ist.

**6.** Verfahren nach Anspruch 1, worin die aromatische Nitroverbindung Nitrobenzol ist.

**7.** Verfahren nach einem oder mehreren der Ansprüche 1 bis 6, worin das Edelmetall der Gruppe VIII Palladium, Ruthenium, Rhodium, Platin oder Iridium ist.

**8.** Verfahren nach einem oder mehreren der Ansprüche 1 bis 7, worin ein zweizähniges Phosphin verwendet wird.

**9.** Verfahren nach Anspruch 8, worin das zweizähnige Phosphin 1,3-Di(diphenylphosphino)propan ist.

**10.** Verfahren nach einem oder mehreren der Ansprüche 1 und 7 bis 9, worin die Säure eine Carbonsäure mit 2 bis 10 Kohlenstoffatomen ist.

**11.** Verfahren nach Anspruch 10, worin die Carbonsäure Essigsäure ist.

**12.** Verfahren nach Anspruch 1, 10 oder 11, worin das Übergangsmetallsalz ein Kupfersalz, ein Eisensalz oder ein Nickelsalz ist.

**13.** Verfahren nach einem der vorangehenden Ansprüche, worin die Reaktion in Gegenwart eines Lösungsmittels stattfindet.

**14.** Verfahren nach Anspruch 13, worin das Lösungsmittel Diethylenglykol-dimethylether oder Nitrobenzol ist.

**15.** Verfahren nach einem der vorangehenden Ansprüche, worin die Reaktionstemperatur zwischen 50° C und 200° C, vorzugsweise zwischen 70° C und 150° C beträgt.

**16.** Katalysatorsystem, bestehend aus
(a) einem Edelmetall der Gruppe VIII oder einer Verbindung davon,
(b) einem zweizähnigen Liganden der Formel

$R^1R^2$-M-A-M-$R^3R^4$

in der M für P, As oder Sb steht, A eine zweiwertige organische brückenbildende Gruppe mit mindestens 2 Kohlenstoffatomen in der Brück ist, wobei keines dieser Kohlenstoffatome Substituenten trägt, die sterische Hinderungen verursachen können, und $R^1$, $R^2$, $R^3$ und $R^4$ gleiche oder verschiedene, gegebenenfalls substituierte, Kohlenwasserstoffgruppen bedeuten und
(c) einem Kupfer-, Nickel- oder Eisensalz einer Säure mit einem pKa von mehr als 2.

**17.** Katalysatorsystem nach Anspruch 16, worin das Edelmetall der Gruppe VIII, Palladium, Ruthenium, Rhodium, Platin oder Tridium ist.

**18.** Katalysatorsystem nach Anspruch 16 oder 17, worin in der Formel des zweizähnigen Liganden $R^1$, $R^2$, $R^3$ und $R^4$ Phenylgruppen bedeuten und M ein Phosphoratom ist und A für eine zweiwertige geradkettige Alkylkette steht.

**19.** Katalysatorsystem nach einem oder mehreren der Ansprüche 16 bis 18, worin die Säure eine Carbonsäure mit 2 bis 10 Kohlenstoffatomen ist.